# EUROPEAN PATENT SPECIFICATION

(11) **EP 4 493 146 B1**
(45) Date of publication and mention of the grant of the patent: **05.11.2025**
(21) Application number: 23710920.2
(22) Date of filing: 16.03.2023
(51) Int. Cl.: A61K 8/34, A61K 8/49, A61K 8/67, A61K 8/73, A61Q 19/02

(54) **A SKIN BRIGHTENING COMPOSITION**
HAUTAUFHELLUNGSZUSAMMENSETZUNG
COMPOSITION POUR ÉCLAIRCIR LA PEAU

(30) Priority: 18.03.2022 EP 22163113
(43) Date of publication of application: 22.01.2025
(73) Proprietor: Unilever IP Holdings B.V., 6708 WH Wageningen (NL); Unilever Global IP Limited, Wirral, Merseyside CH62 4ZD (GB)
(72) Inventor: BETADPUR, Anagha, 6708 WH Wageningen (NL); DUTTA, Maitreyee, 6708 WH Wageningen (NL); NAIR, Nirmala, Santosh, 6708 WH Wageningen (NL)
(74) Representative: James, Helen Sarah
(86) International application number: PCT/EP2023/056708
(87) International publication number: WO 2023/175061

(56) References cited:
- WO-A1-2021/078611
- CN-A- 107 412 045
- US-B2- 10 500 152
- US-B2- 10 952 952
- DATABASE GNPD [online] MINTEL; 27 July 2018 (2018-07-27), ANONYMOUS: "Bright Biotic Dark Spot Minimizing Serum", XP055963762, retrieved from https://www.gnpd.com/sinatra/recordpage/5804013/ Database accession no. 5804013

## Description

### Field of the Invention

The present invention relates to a personal care composition which brightens skin. It more particularly relates to a topical composition which is effective against hyperpigmentation especially that caused by exposure to the sun.

### Background of the Invention

Most people consider skin appearance especially on the face as one of the key indicators of their own beauty and health. Having an even skin tone which is bright and free of blemishes is thus desired by many. The degree and evenness of pigmentation of the skin is affected by factors like age, hormonal changes, occurrence of acne and exposure to sunlight and pollution. These can cause spots or freckles, hyper-pigmentation on certain localised areas of skin and also under-eye dark circles. Many people believe that certain life-style factors like hydration (quantity of water consumed), the type of food and quantity and quality of sleep also have an effect on skin appearance including the dark circles under the eyes. Since people are aware that changes like leading a healthier lifestyle may take a long time for evening out skin appearance, they rely on cosmetic solution to give them temporary solution to blemishes on their skin. They also seek such products to reduce the skin darkening caused by exposure to sunlight. To meet this need, many attempts have been made to develop products that reduce the pigment production in the melanocytes. Reference is made to the "Bright Biotic Dark Spot Minimizing Serum" (Mintel ID no. 5804013) comprising niacinamide, hexylresorcinol and Inulin.

The present inventors have been working in this area for a long time and have also filed several patent applications and produced various cosmetic products which are in the market. In the present invention, they were specifically looking for a highly effective solution to this problem which could also be used as a general cosmetic product for delivering even and bright skin tone especially on exposed skin surfaces.

They want to ensure that the product developed is as effective, if not better than the most effective product in the market. After screening a large number of actives they found that one or more of a skin care active known for providing even skin tone like alkyl resorcinol or a carboxylic acid functionalised heteroaromatic compound or a retinoic acid compound known for anti-aging benefit when combined with inulin provides for enhanced skin brightening and even skin tone. To the knowledge of the present inventors such a combination of actives to deliver this benefit is not known before. Further, it is desirable that conventional skin care actives known for this benefit like alkyl resorcinol or a carboxylic acid functionalised heteroaromatic compound or a retinoic acid compound are generally included in lower quantities and it not only reduces cost but also ensures better compatibility with other ingredients in the formulation thereby leading to better formulation stability. The present inventors were looking for a solution where an additive could be identified that synergises with the above conventional skin care actives such that these actives could be included at very low concentrations, thereby providing for all of the above advantages.

It is thus an object of the present invention to provide for a solution to the problem of hyperpigmentation and uneven skin appearance thereby delivering a brighter skin appearance.

It is another object of the present invention to provide such a solution which involves inclusion of lower amounts of the skin care actives thereby ensuring better formulation stability.

### Summary of the Invention

The first aspect of the present invention relates to a personal care composition comprising:
(i) a retinoic acid precursor selected from one or more of retinyl esters, retinol, retinal and retinoic acid; (ii) a carboxylic acid functionalized heteroaromatic compound selected from one or more of nicotinic acid, picolinic acid, nicotinate, niacinamide, and picolinamide; and (iii) an alkyl resorcinol; (iv)lnulin; and (v) a cosmetically acceptable vehicle

Another aspect of the present invention relates to a method of providing brightness to skin, comprising the step of applying a composition of the first aspect on to skin.

### Detailed Description of the Invention

These and other aspects, features and advantages will become apparent to those of ordinary skill in the art from a reading of the following detailed description and the appended claims. For the avoidance of doubt, any feature of one aspect of the present invention may be utilised in any other aspect of the invention. The word "comprising" is intended to mean "including" but not necessarily "consisting of" or "composed of." In other words, the listed steps or options need not be exhaustive. It is noted that the examples given in the description below are intended to clarify the invention and are not intended to limit the invention to those examples per se. Similarly, all percentages are weight/weight percentages unless otherwise indicated. Except in the operating and comparative examples, or where otherwise explicitly indicated, all numbers in this description and claims indicating amounts of material or conditions of reaction, physical properties of materials and/or use are to be understood as modified by the word "about". Numerical ranges expressed in the format "from x to y" are understood to include x and y. When for a specific feature multiple preferred ranges are described in the format "from x to y", it is understood that all ranges combining the different endpoints are also contemplated.

The composition of the invention is meant to be used for personal care or for cosmetic use and could also be referred to as a personal care composition or a cosmetic composition. By a "personal care composition" as used herein, is meant to include a composition for topical application i.e external surfaces of the skin of humans. Such a composition may be classified as leave-on or rinse off, and includes any product applied to a human body for improving appearance, cleansing, odour control or general aesthetics. The composition is preferably of the leave-on type. The composition of the present invention can be in the form of a liquid, lotion, cream, foam, stick, serum, essence or gel. Preferred compositions include leave-on gels, lotions, serum or creams, preferably it is in the serum or cream form.

"Skin" as used herein is meant to include skin on the face and body (e.g., neck, chest, back, arms, underarms, hands, legs and scalp) and especially to the exposed parts thereof.

The present invention provides for a personal care composition comprising (i) a retinoic acid precursor; (ii) a carboxylic acid functionalized heteroaromatic compound selected from one or more of nicotinic acid, picolinic acid, nicotinate, niacinamide, and picolinamide; (iii) an alkyl resorcinol; (iv) Inulin; and (v) a cosmetically acceptable vehicle.

The retinoic acid precursor as per this invention, may be selected from one or more of retinyl esters, retinol, retinal and retinoic acid, preferably retinal or retinyl ester. Examples of retinyl esters suitable for use in the invention include but are not limited to: retinyl palmitate, retinyl formate, retinyl acetate, retinyl propionate, retinyl butyrate, retinyl valerate, retinyl isovalerate, retinyl hexanoate, retinyl heptanoate, retinyl octanoate, retinyl nonanoate, retinyl decanoate, retinyl undecandate, retinyl taurate, retinyl tridecanoate, retinyl myristate, retinyl pentadecanoate, retinyl heptadeconoate, retinyl stearate, retinyl isostearate, retinyl nonadecanoate, retinyl arachidonate, retinyl behenate, retinyl linoleate, and retinyl oleate. The preferred ester for use in the present invention is selected from retinyl palmitate, retinyl acetate, retinyl propionate and mixtures thereof. Retinyl linoleate and retinyl oleate are also preferred. The most preferred retinoic acid precursor is retinyl propionate. Retinoic acid precursor is employed in the inventive composition in an amount from 0.001 to 10%, preferably in an amount from 0.01 to 1%, most preferably in an amount from 0.01 to 0.5%.

The composition of the invention may comprise a carboxylic acid functionalized heteroaromatic compound. The carboxylic acid functionalized heteroaromatic compound may be selected from one or more of nicotinic acid, picolinic acid, nicotinate, niacinamide, and picolinamide. It is preferably one or both of niacinamide and picolinamide, most preferably niacinamide. The carboxylic acid functionalized heteroaromatic compound is typically included in an amount of 0.01 to 10%, preferably, 0.05 to 7%, and, more preferably, 0.1 to 5% by weight of the cosmetic composition, including all ranges subsumed therein.

Resorcinol is a dihydroxy phenol compound (i.e., 1,3-dihydroxybenzene) characterized by alcohol groups (-OH) at positions 1 and 3. The chemical structure of resorcinols may be modified, resulting in substituted resorcinols characterized by at least one substituent in the 2, 4, 5 or 6 position. It is preferred that the resorcinol comprises at least one substituent comprising 2 to 11 carbon atoms, preferably, 2 to 8 carbon atoms, most preferably 2 to 6 carbon atoms. It is particularly preferred that at least one substituent comprises an alkyl group, more preferably, an unsaturated alkyl group. The resorcinol compounds comprised in the cosmetic compositions of the present invention are oil-soluble alkyl resorcinols

Preferably, the resorcinol of the cosmetic composition is only substituted at position 4 with an alkyl group, i.e., the resorcinol is an alkylresorcinol, more preferably, a 4-substituted alkylresorcinol. Illustrated but not limiting examples of 4-substituted alkylresorcinols include 4-ethyl resorcinol, 4-hexylresorcinol, 4-phenylethylresorcinol, 4-cyclopentyl resorcinol, 4-cyclohexylresorcinol, 4-octylresorcinol, and mixtures thereof. The more preferred alkyl resorcinol for use in the present invention is one or both of 4-ethyl resorcinol and 4-hexyl resorcinol; most preferred being 4-hexyl resorcinol. The composition comprises 0.001 to 10%, preferably, 0.01 to 5%, and, most preferably, 0.01 to 1% alkylresorcinol by weight of the cosmetic composition, including all ranges subsumed therein.

Inulin is a polysaccharide which is built up of 2-60 fructose units with one terminal glucose molecule. Inulin is the generic name covering all β-(2,1) fructans. Inulin has the general structure as given below.

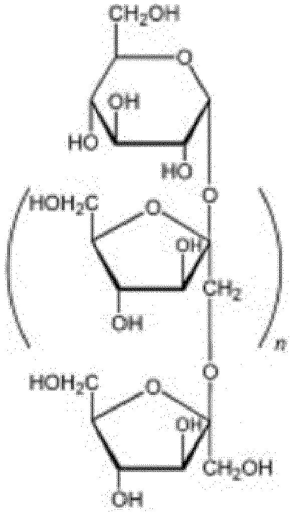

Inulin occurs naturally in many common plants such as chicory, Jerusalem artichoke, onion, garlic, barley, rye and wheat. It is present in high quantities in chicory and is generally extracted from it for commercial production. It is a well known prebiotic with antimicrobial and anticancer activity. Inulin is accepted in most countries as a food ingredients that can be used without restrictions in food formulations. It is present in skin care compositions as humectants and moisturizing agents. It has the CAS number 9005-80-5. It has the IUPAC name fructosane. Inulin is preferably included in the composition of the present invention in 0.01 to 10%, more preferably 0.01 to 5%, most preferably 0.01 to 1% by weight of the composition. For use in the present invention, inulin was procured from Sigma Aldrich, USA.

The composition preferably comprises an oil which has a Hansen total solubility parameter (δₜ) value within the range of 16.5 to 22. The preferred oils may be selected from one or more of isopropyl myristate, isopropyl palmitate, caprylic/capric triglycerides and benzyl alcohol. These oils are preferably included in an amount of 8 to 25% by weight of the composition.

An emulsifying polymer is preferably included in the composition of the invention. The emulsifying polymer is preferably a swellable polymer powder made of pure acrylic acid monomers or a mixture of acrylic acid and methacrylic acid monomers, and are, preferably, polymers having a chemical name of Acrylates/C₁₀-₃₀ Alkyl Acrylate Crosspolymer, Carbomer, or a mixture thereof. In one aspect, the polymer is a crosslinked or non-crosslinked homopolymer. In another aspect, the polymer is a crosslinked or non-crosslinked copolymer. The emulsifying polymer is multifunctional and is capable of emulsifying, stabilizing, and/or building viscosity of a composition. It is within the scope of the present compositions to employ a polymeric emulsifier alone or in combination with other additional polymeric emulsifiers. Illustrative but nonlimiting Acrylates/C₁₀-₃₀ Alkyl Acrylate Crosspolymer for use in the present cosmetic compositions are made commercially available by the supplier The Lubrizol Corporation under such trade names as PEMULEN^{™} TR-1, PEMULEN^{™} TR-2, PEMULEN^{™} EZ-4U, CARBOPOL^{®} Ultrez 20, CARBOPOL^{®} Ultrez 21, CARBOPOL^{®} 1382 and CARBOPOL^{®} ETD 2020. Desirable Carbomers for use in the present composition include CARBOPOL^{®} Ultrez 10 and CARBOPOL^{®} 980, both commercially sold by The Lubrizol Corporation. Emulsifying polymers are included in the cosmetic composition in an amount of 0.01 to 5%, preferably, from 0.02 to 4%, and, more preferably, from 0.03 to 3%, by total weight of the composition. It is still more preferred that the polymers are included in the cosmetic composition at 0.04 to 2% by weight, most preferably, 0.05 to 1% by weight of the cosmetic composition, including all ranges subsumed therein.

The composition of the invention may additionally comprise one or more of a sunscreen, photostabilizer, skin-brightening agent, wrinkle-reducing agent, and coloring agent.

The composition may additionally comprise an organic sunscreen selected from one or both of a UVA sunscreen and a UVB sunscreen. Sunscreens include those materials commonly employed to block ultraviolet light. Illustrative compounds are the derivatives of PABA, cinnamate and salicylate. For example, avobenzophenone (Parsol 1789^{®}) octyl methoxycinnamate and 2-hydroxy-4-methoxy benzophenone (also known as oxybenzone) can be used. Octyl methoxycinnamate and 2-hydroxy-4-methoxy benzophenone are commercially available under the trademarks, Parsol MCX and Benzophenone-3, respectively. The exact amount of sunscreen employed in the compositions can vary depending upon the degree of protection desired from the sun's UV radiation. Additives that reflect or scatter the sun rays may also be employed. These additives include oxides like zinc oxide and titanium dioxide.

The composition of the present invention comprises a cosmetically acceptable vehicle, which may act as diluents, dispersants. and/or carriers for the actives used in the composition, so as to facilitate their distribution when the composition is applied to the skin. The cosmetically acceptable vehicle suitable for use in the present invention may be aqueous, anhydrous or an emulsion; preferably aqueous or an emulsion, with the emulsion being a water-in-oil or oil-in-water emulsion, the latter being most preferred. Water when present typically makes up the balance of the composition. Preferably water is present in a concentration of 5 to 99%, more preferably from 20 to 80%, still more preferably from 40 and 80% by weight of the composition.

The composition of the present invention may be delivered in a serum, cream, lotion or gel form preferably in cream form. A preferred format for the solid form of the composition is a cream, furthermore preferably one which has a vanishing cream base. Vanishing cream base is one which comprises 3 to 25 wt% fatty acid. Optionally, the composition may comprise 0.1 to 10 wt% soap. When included, the fatty acid is preferably a C10 to C22 fatty acid, more preferably a C16 to C18 fatty acid. Most preferably the fatty acids are stearic acid or palmitic acid or a mixture thereof and the soap is preferably the potassium salt of the fatty acid mixture. The fatty acid is often hystric acid which is substantially (generally about 90 to 95 %) a mixture of 45 % stearic acid and 55 % palmitic acid. The most preferred cream is one having 3 to 25 wt% fatty acid and 0.1 to 10 wt% soap.

Preferably, the composition comprises emollients. Examples of emollients that may be used in the leave-on composition include stearyl alcohol, glyceryl monoricinoleate, mink oil, isopropyl isostearate, isobutyl palmitate, isocetyl stearate, oleyl alcohol, isopropyl laurate, hexyl laurate, decyl oleate, octadecan-2-ol, isocetyl alcohol, eicosanyl alcohol, behenyl alcohol, cetyl palmitate, silicone oils such as dimethylpolysiloxane, din-butyl sebacate, isopropyl myristate, isopropyl palmitate, isopropyl stearate, butyl stearate, polyethylene glycol, triethylene glycol, lanolin, cocoa butter, corn oil, cotton seed oil, olive oil, palm kernel oil, rape seed oil, safflower seed oil, evening primrose oil, soybean oil, sunflower seed oil, avocado oil, sesame seed oil, coconut oil, arachis oil, castor oil, acetylated lanolin alcohols, petroleum jelly, mineral oil, butyl myristate, isopropyl linoleate, lauryl lactate, myristyl lactate, decyl oleate, myristyl myristate and mixtures thereof.

Preferably, the composition comprises solvents. Examples of solvents that may be used in the composition include ethyl alcohol, isopropanol, acetone, ethylene glycol mono ethyl ether, diethylene glycol mono butyl ether, diethylene glycol mono ethyl ether and mixtures thereof. The composition may comprise polyhydric alcohols which may be selected from one or more of glycerine, 1,3-butylene glycol, propylene glycol, 1,3-propanediol, pentylene glycol, hexylene glycol, and sorbitol.

Preferably, the composition comprises powders. Examples of powders that may be used in the composition include chalk, talc, fullers earth, kaolin, starch, gums, colloidal silica sodium polyacrylate, tetra alkyl and/or trialkyl aryl ammonium smectites, chemically modified magnesium aluminium silicate, organically modified montmorillonite clay, hydrated aluminium silicate, fumed silica, carboxyvinyl polymer, sodium carboxymethyl cellulose, ethylene glycol monostearate and mixtures thereof.

Preferably, the composition comprises preservatives to protect against the growth of potentially harmful microorganisms. Examples of ingredients that may be used as preservatives in the composition include alkyl esters of para-hydroxybenzoic acid, hydantoin derivatives, propionate salts, and a variety of quaternary ammonium compounds. More preferably, ingredients that may be used as preservative in the composition are sodium benzoate, iodopropynyl butyl carbamate, methylisothiazolinone, iodopropynylbutylcarbamate, phenoxyethanol, methyl paraben, propyl paraben, imidazolidinyl urea, sodium dehydroacetate, ethylhexylglycerin, benzyl alcohol, alkane diols and mixtures thereof. When present in the composition, preservatives are added preferably in an amount 0.001 to 5 wt%, more preferably 0.01 to 3 wt% and most preferably 0.02 to 2 wt%, even most preferably 0.25 to 1.5%.

Preferably, the composition comprises a range of other optional ingredients that include antioxidants, binders, buffering agents, colorants, astringents, fragrance, opacifying agents, conditioners, exfoliating agents, pH adjusters, skin sensates, skin soothing agents, and skin healing agents.

It is within the scope of the present invention that the compositions claimed here are ecofriendly and therefore are free of certain ingredients that are increasingly being implicated in long term detriment to the environment. Thus, one aspect of the present invention relates to a composition which is free of sulphate surfactants. Another aspect relates to compositions herein that are free of preservatives. Yet another aspect relates to compositions that are free of acrylate polymers. Also within the scope of the present invention are composition which are free of titanium dioxide. Green (i.e. biodegradable chelating agents are preferred) for use in such compositions i.e the composition is preferably free of conventional chelating agents like EDTA. By "free of" a certain ingredient, as per this present invention is meant that the composition comprises less than 0.1, preferably less than 0.05, furthermore preferably less than 0.01% of the ingredient by weight of the composition. They are optimally absent from the composition.

The packaging for the composition of this invention can be a patch, bottle, tube, roll-ball applicator, propellant driven aerosol device, squeeze container or lidded jar.

In an alternate aspect, the invention relates to use of the composition according to the invention for skin brightening.

In yet another aspect, the invention relates to a method of brightening the skin of a human, the method comprising the step of applying the composition according to the invention onto the skin.

Also described is a method of providing microbiome benefit to skin comprising the step of applying a composition according to the present invention on to skin.

Inulin has been known as a prebiotic to deliver beneficial effects on the gut microbiome. It stimulates the growth and activities of health promoting microorganisms while inhibiting enteropathogenic bacteria. The beneficial microorganisms ferment inulin and produces acids including short-chain fatty acids that lower the pH in the colon and inhibit pathogens. The present inventors have found that inclusion of inulin in the topical composition of the present invention, helps generate beneficial metabolites which in turn can promote skin health, for example, S.epidermidis fermented Inulin can deliver depigmentation benefits.

The invention will now be illustrated by means of the following non-limiting examples.

### Examples

### Examples A-F, 1,2: %Cellular melanin content of various combinations of actives vs. Control

Various samples containing actives were prepared and the % cellular melanin content was measured. The protocol for measuring the cellular melanin is summarized below:

### Cell cultures:

Neonatal primary human epidermal melanocytes were sourced from Life Technologies. Melanocytes were maintained in Medium 254 supplemented with human melanocyte growth supplement (MGM). HaCaT keratinocytes were maintained in DMEM medium containing 10% heat inactivated FBS. All media were also supplemented with 10 U/mL penicillin G and 0.1 mg/mL streptomycin sulphate. Both cell types were maintained at 37°C in a humidified incubator with 5% CO₂ atmosphere. Maintenance and sub-culturing of cells were carried out as per the manufacturer's instructions.

For setup of co-cultures, 40,000 cells each of HaCaT keratinocytes and melanocytes were mixed in 1 mL of a 1:1 mix of MGM: KGM (KGM: Epilife medium supplemented with human keratinocyte growth supplement) and seeded as such per well, in 12 well Nunc plates.

### Active addition:

Actives were prepared in media and test concentrations were added to cells along with relevant controls 24 h post seeding. After 72 h of active treatment, cell viability (using calcein method) and measurement of cellular melanin content was determined as described below.

### Cell Viability assay:

Briefly, spent media was removed and cells washed once with 0.4 mL of Phosphate buffered saline. Fresh 1 µM calcein- acetoxymethyl in phosphate buffered saline was added to each well. Plates were incubated for 30 min. at 37°C under 5% CO₂ incubator. Calcein fluorescence was measured (excitation at 490 nm and emission at 520 nm) using TECAN M1000 plate reader.

### Melanin content assay:

After calcein fluorescence readings were obtained, cells were drained and fresh 0.15 mL of 1N NaOH (in 10% DMSO) per well was added. Cells were lysed by resuspension and incubation (60°C for 1 h). Then, 0.1 mL of this lysate was transferred to a fresh 384-well plate and OD was measured at 405nm in a TECAN M1000 plate reader. Melanin content was calculated as percentage cellular melanin vs control.

The following samples as given in Table -1 were prepared and the % melanin content of each as measured, are also given in the table.

**Table - 1**

| Example | Actives used (wt%) | % cellular melanin |
|---|---|---|
| A | Control | 100 |
| B | Inulin (0.125%) | 86 |
| C | Inulin (0.0625%) | 96 |
| D | DMSO | 100 |
| E | 4-hexyl resorcinol (0.0002%) | 69* |
| F | COM1 | 97 |
| 1 | COM1 + Inulin (0.125%) | 74^{*#} |
| 2 | COM1 + Inulin (0.0625%) | 70^{*#$} |

| | | |
|---|---|---|
| COM1 refers to a combination of Niacinamide (0.0122%) + 4-hexyl resorcinol (0.00002%) + retinyl propionate (0.00003%) *refers to p<0.05 vs Example D # refers to p<0.05 vs. Example F $ refers to p<0.05 vs Example C | | |

The data in the table -1 above indicates that compositions as per the invention (Examples 1 and 2) provide significantly superior reduction in cellular melanin content as compared to the respective individual ingredients. The result for Example -2 is comparable to a very effective positive control (Example E).

It is to be understood that the experiments described above were conducted in an invitro assay to evaluate % melanin at the cellular level. It is expected that the concentrations to be actually used to prepare a composition for topical use would be vastly different. The concentrations is generally orders of magnitude higher due to the following reasons that affect the difference in concentration in the bulk as compared to that at the cellular level. The composition may be formulated as an emulsion or a gel with very many additional ingredients. The concentration of the desired actives in the oil phase and in the water phase, is expected to be very different. They may also have very different physical and hydrodynamic properties like partition coefficients, diffusional rates, convective transport rates, rheological properties etc. Therefore, it is expected that the concentrations to be used when formulated as a composition would be very different, often orders of magnitude higher, from that at the cellular level, which is the concentration at which the experiments were carried out.

## Claims

1. A personal care composition comprising:
(a) a retinoic acid precursor selected from one or more of retinyl esters, retinol, retinal and retinoic acid;
(b) a carboxylic acid functionalized heteroaromatic compound selected from one or more of nicotinic acid, picolinic acid, nicotinate, niacinamide, and picolinamide;
(c) an alkyl resorcinol ;
(d) Inulin; and
(e) a cosmetically acceptable vehicle.

2. A composition as claimed in claim 1 comprising 0.001 to 10% by weight of the retinoic acid precursor.

3. A composition as claimed in claim 1 or 2, comprising 0.01 to 10% by weight of the carboxylic acid functionalized heteroaromatic compound.

4. A composition as claimed in any one of the preceding claims, wherein the alkylresorcinol comprises one or more of 4-hexylresorcinol, 4-ethylresorcinol, 4-phenylethylresorcinol, 4-cyclopentylresorcinol, 4-cyclohexylresorcinol, and 4-octylresorcinol.

5. A composition as claimed in any one of the preceding claims comprising 0.001 to 10% by weight of alkylresorcinol.

6. A composition as claimed in any one of the preceding claims comprising 0.01 to 10% inulin.

7. A composition as claimed in any one of the preceding claims additionally comprising an oil preferably selected from one or more of isopropyl myristate, isopropyl palmitate, caprylic/capric triglycerides and benzyl alcohol.

8. A composition as claimed in any one of the preceding claims additionally comprising an emulsifying polymer selected from one or more of an acrylates/C₁₀-₃₀ alkyl acrylate crosspolymer, and carbomer preferably included in 0.01 to 5% by weight of the composition.

9. A composition as claimed in any one of the preceding claims in serum, cream, lotion or gel form preferably in serum or cream form.

10. A method of providing brightness to skin along with an even skin tone comprising the step of applying a composition as claimed in any one of the preceding claims on to skin.

## Patentansprüche

1. Körperpflegezusammensetzung, die umfasst:
(a) einen Retinsäure-Vorläufer, ausgewählt unter einer oder mehreren der Verbindungen Retinylestern, Retinol, Retinal und Retinsäure;
(b) eine Carbonsäure-funktionalisierte heteroaromatische Verbindung, ausgewählt unter einer oder mehreren der Verbindungen Nicotinsäure, Picolinsäure, Nicotinat, Niacinamid und Picolinamid;
(c) ein Alkylresorcin;
(d) Inulin; und
(e) ein kosmetisch akzeptables Trägermittel.

2. Zusammensetzung nach Anspruch 1, die 0,001 bis 10 Gew.-% des Retinsäure-Vorläufers umfasst.

3. Zusammensetzung, wie im Anspruch 1 oder 2 beansprucht, die 0,01 bis 10 Gew.-% der Carbonsäure-funktionalisierten heteroaromatischen Verbindung umfasst.

4. Zusammensetzung, wie in irgendeinem der vorhergehenden Ansprüche beansprucht, wobei das Alkylresorcin eine oder mehrere der Verbindungen 4-Hexylresorcin, 4-Ethylresorcin, 4-Phenylethylresorcin, 4-Cyclopentylresorcin, 4-Cyclohexylresorcin und 4-Octylresorcin umfasst.

5. Zusammensetzung, wie in irgendeinem der vorhergehenden Ansprüche beansprucht, die 0,001 bis 10 Gew.-% Alkylresorcin umfasst.

6. Zusammensetzung, wie in irgendeinem der vorhergehenden Ansprüche beansprucht, die 0,01 bis 10% Inulin umfasst.

7. Zusammensetzung, wie in irgendeinem der vorhergehenden Ansprüche beansprucht, die zusätzlich ein Öl umfasst, das vorzugsweise unter einer oder mehreren der Verbindungen Isopropylmyristat, Isopropylpalmitat, Capryl-/ Caprinsäure-triglyceriden und Benzylalkohol ausgewählt ist.

8. Zusammensetzung, wie in irgendeinem der vorhergehenden Ansprüche beansprucht, die zusätzlich ein emulgierendes Polymer umfasst, das unter einer oder mehreren der Verbindungen Acrylate/C₁₀-₃₀-Alkylacrylat-Kreuzpolymer und Carbomer, vorzugsweise einbezogen in einer Menge von 0,01 bis 5 Gew.-% der Zusammensetzung, ausgewählt ist.

9. Zusammensetzung, wie in irgendeinem der vorhergehenden Ansprüche beansprucht, in Form eines Serums, einer Creme, einer Lotion oder eines Gels, vorzugsweise in Form eines Serums oder einer Creme.

10. Verfahren zur Verleihung von Aufhellung zusammen mit einem gleichmäßigen Hautton, umfassend den Schritt des Aufbringens einer Zusammensetzung, wie in irgendeinem der vorhergehenden Ansprüche beansprucht, auf die Haut.

## Revendications

1. Composition de soin personnel comprenant :
(a) un précurseur d'acide rétinoïque qui est un ou plusieurs choisis parmi les esters rétinyliques, le rétinol, le rétinal et l'acide rétinoïque ;
(b) un composé hétéroaromatique à fonctionnalisation acide carboxylique qui est un ou plusieurs choisis parmi l'acide nicotinique, l'acide picolinique, un nicotinate, le niacinamide, et le picolinamide ;
(c) un alkylrésorcinol ;
(d) de l'inuline ; et
(e) un véhicule acceptable en cosmétique.

2. Composition selon la revendication 1, comprenant 0,001 à 10 % en poids du précurseur d'acide rétinoïque.

3. Composition selon la revendication 1 ou 2, comprenant 0,01 à 10 % en poids du composé hétéroaromatique à fonctionnalisation acide carboxylique.

4. Composition selon l'une quelconque des revendications précédentes, dans laquelle l'alkylrésorcinol comprend un ou plusieurs parmi le 4-hexylrésorcinol, le 4-éthylrésorcinol, le 4-phényléthylrésorcinol, le 4-cyclopentylrésorcinol, le 4-cyclohexylrésorcinol, et le 4-octylrésorcinol.

5. Composition selon l'une quelconque des revendications précédentes, comprenant 0,001 à 10 % en poids d'alkylrésorcinol.

6. Composition selon l'une quelconque des revendications précédentes, comprenant 0,01 à 10 % d'inuline.

7. Composition selon l'une quelconque des revendications précédentes, comprenant de plus une huile qui est de préférence une ou plusieurs choisies parmi le myristate d'isopropyle, le palmitate d'isopropyle, les triglycérides capryliques/capriques, et l'alcool benzylique.

8. Composition selon l'une quelconque des revendications précédentes, comprenant de plus un polymère émulsifiant qui est un ou plusieurs choisis parmi un copolymère réticulé d'acrylates/acrylate d'alkyle en C₁₀ à C₃₀, et un carbomère de préférence incorporé à raison de 0,01 à 5 % en poids de la composition.

9. Composition selon l'une quelconque des revendications précédentes, sous forme de sérum, crème, lotion ou gel, de préférence sous forme de sérum ou de crème.

10. Procédé pour conférer de l'éclat à une peau conjointement avec un teint unifié, comprenant l'étape d'application sur la peau d'une composition selon l'une quelconque des revendications précédentes.
